(19) Europäisches Patentamt

European Patent Office

Office européen des brevets

(11) **EP 1 723 901 A1**

(12) **EUROPEAN PATENT APPLICATION**

(43) Date of publication:
**22.11.2006 Bulletin 2006/47**

(51) Int Cl.:
*A61B 3/113* (2006.01)

(21) Application number: **06075964.4**

(22) Date of filing: **28.04.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **16.05.2005 US 130360**

(71) Applicant: **Delphi Technologies, Inc.
Troy, MI 48007 (US)**

(72) Inventors:
• **Smith, Matthew R
Westfield, IN 46074 (US)**

• **Witt, Gerald J.
Carmel, IN 46032 (US)**
• **Zhang, Harry
Carmel, IN 46033 (US)**
• **Harter, Joseph E. Jr.
Kokomo, IN 46902 (US)**
• **Scharenbroch, Gregory K.
Kokomo, IN 46901 (US)**

(74) Representative: **Denton, Michael John et al
Delphi European Headquarters,
64 avenue de la Plaine de France,
Paris Nord II,
B.P. 65059, Tremblay en France
95972 Roissy Charles de Gaulle Cedex (FR)**

(54) **Vehicle operator monitoring system and method**

(57)    A vehicle operator monitoring method (100) is disclosed. The method (100) includes the steps of capturing (104-128) a series of ocular profiles (50a, 50b, 50c) of a vehicle operator (20), conducting an analysis (130) of the series of ocular profiles (50a, 50b, 50c) of the vehicle operator (20), and applying the analysis (132) to a vehicle system (250) to maintain or adjust a state of the vehicle system (250). The vehicle system (250) may be an automated cruise control system or a forward collision warning system.

Fig.2.

# Description

TECHNICAL FIELD

The disclosure relates to vehicle operator monitoring systems. More specifically, the disclosure relates to vehicle operator attentiveness imaging for adjusting vehicle systems.

BACKGROUND OF THE INVENTION

Each year numerous automobile accidents are caused by vehicle operator distractions. The National Highway Traffic Safety Administration (NHTSA) estimates that vehicle operator distraction is directly involved in twenty to thirty percent of all automobile accidents or roughly 1.6 million automobile accidents in the U.S. annually. Visual distraction of the vehicle operator is attributed to many accidents. A need therefore exists to provide a real-time monitoring of the visual distraction of the vehicle operator.

BRIEF DESCRIPTION OF THE DRAWINGS

The present disclosure is described, by way of example, with reference to the accompanying drawings, in which:

> Figure 1 is a general view of a vehicle operator attentiveness imaging system;
> Figure 2 is an environmental view of the vehicle operator attentiveness imaging system of Figure 1 according to an embodiment;
> Figure 3 is a view of an instrument panel including a vehicle operator attentiveness imaging device of Figure 1 according to an embodiment;
> Figure 4 is an environmental view of a vehicle operator attentiveness imaging system of Figure 1 according to an embodiment;
> Figures 5A-5C illustrate views of a vehicle operator's ocular profiles;
> Figure 6 illustrates a forward view window correlated to the ocular profile of Figure 5A according to an embodiment;
> Figure 7 is a flow diagram illustrating a vehicle operator attentiveness imaging system according to an embodiment;
> Figure 8 illustrates an environmental view of a vehicle employing an automated cruise control system according to an embodiment; and
> Figure 9 is a flow diagram illustrating an automated cruise control system according to an embodiment.

DESCRIPTION OF THE PREFERRED EMBODIMENT

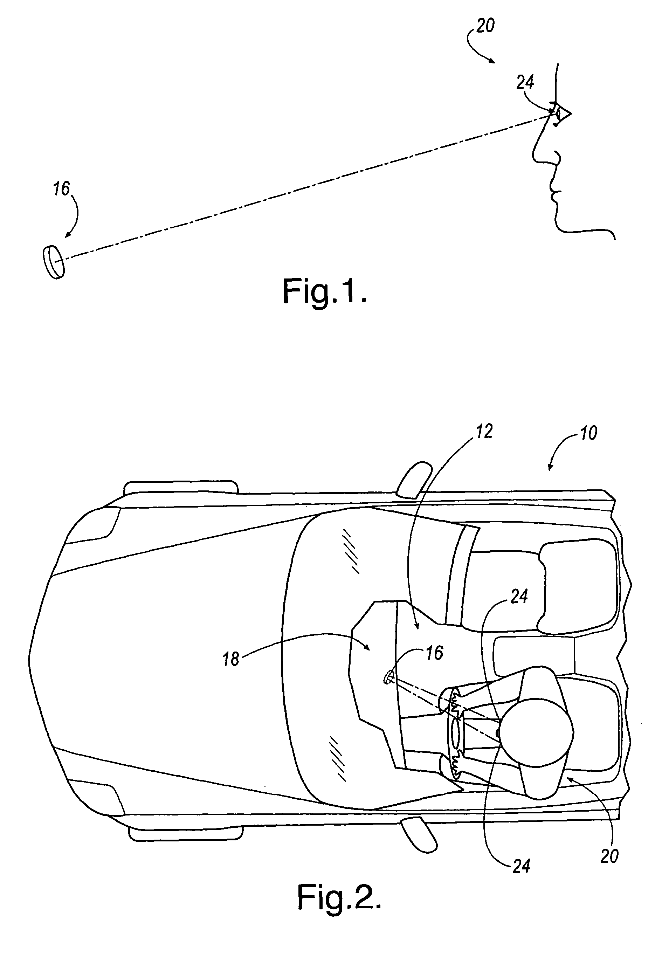

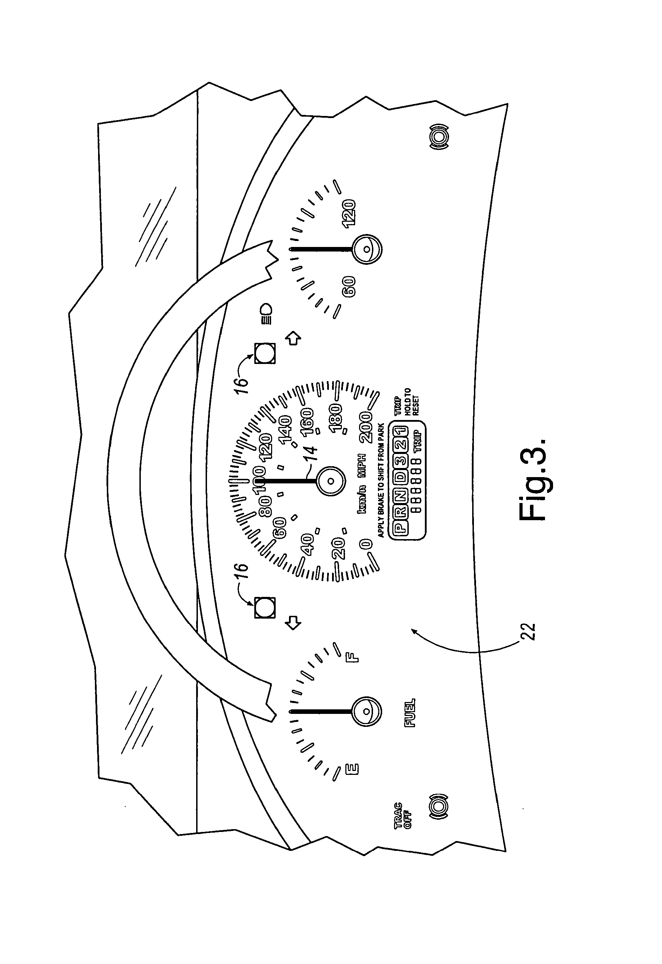

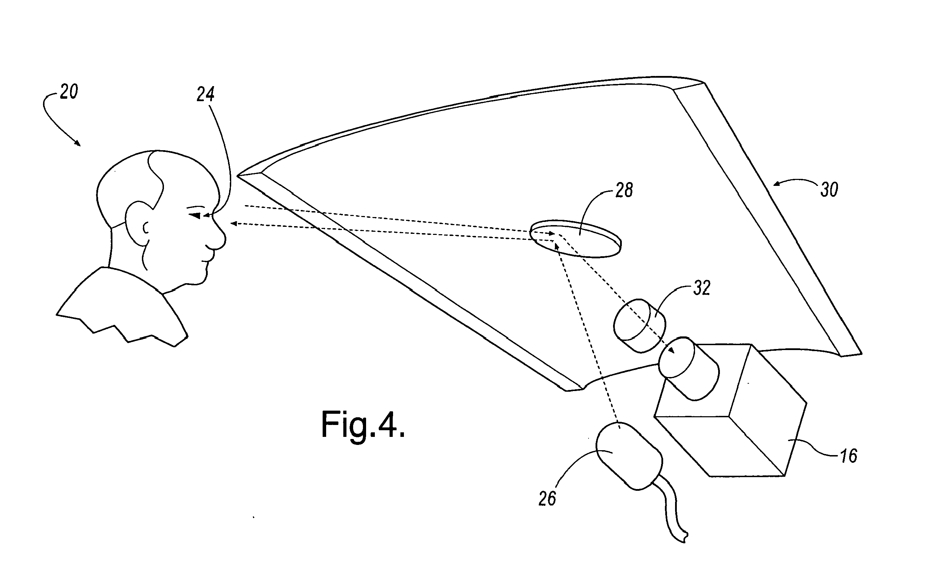

Referring to Figures 1 and 2, a passenger compartment 12 of a vehicle 10 is generally shown equipped with a vehicle operator attentiveness imaging system having a video imaging camera, which is shown generally at 16. The video imaging camera 16 may be positioned in any desirable location, such as, for example, on/within the dashboard/instrument panel 18 for capturing images of eyes 24 of a vehicle operator 20. According to the illustrated embodiment shown in Figure 2, the video imaging camera 16 may be mounted generally on a mid-region of the dashboard 18 in the front region of the passenger compartment 12. Referring to Figure 3, another embodiment may employ a pair of video imaging cameras 16 within an instrument panel cluster 22. Alternatively, as shown in Figure 4, reflected images of the vehicle operator's eyes 24 may be captured with an optical system including a video imaging camera 16, an illuminator 26, a mirror 28 located about an inboard surface of a windshield 30, and, if desired, a band-pass filter 32 to block ambient light that would otherwise saturate the video imaging camera 16.

The video imaging camera 16 may include CCD/CMOS active-pixel digital image sensors mounted as individual chips onto a circuit board (not shown). One example of a CMOS active-pixel digital image sensor is Model No. PB-0330, commercially available from Photobit, which has a resolution of 640H x 480V. The use of digital image sensors for the video imaging camera 16 also allows for the detection of stereo information. The video imaging camera 16 may also be coupled to an eye tracking processor (not shown). The eye tracking processor may include a frame grabber for receiving the video frames generated by the video imaging camera 16. The video imaging camera may also be coupled to a video processor for processing the video frames. The video processor includes memory, such as random access memory (RAM), read-only memory (ROM), and other memory as should be readily apparent to those skilled in the art. Other features of the vehicle operator attentiveness systems of Figures 1-4 are described in application serial numbers 10/103,202; 10/291,913; and 10/986,240 and are under assignment to the assignee of the present disclosure.

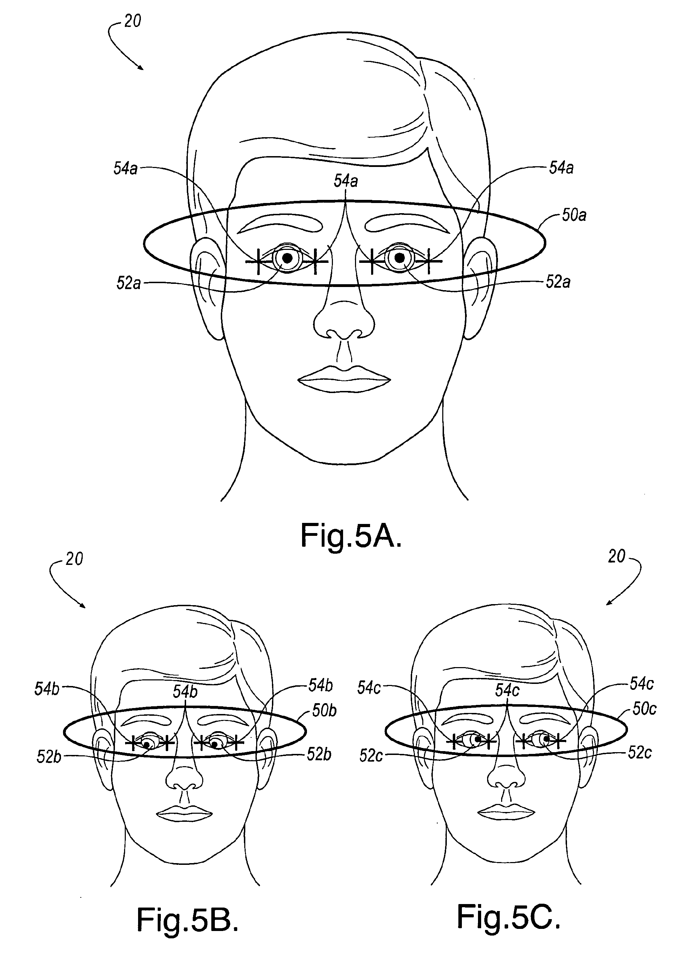

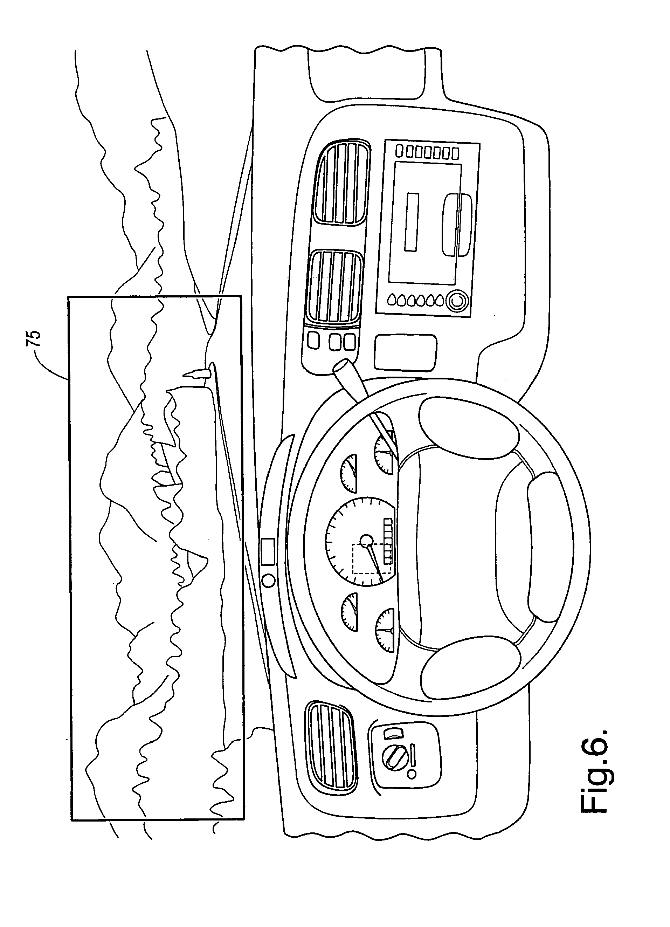

Referring to Figures 5A-5C, the vehicle operator attentiveness imaging systems described in Figures 1-4 may capture ocular profiles. Examples of ocular profiles are shown generally at 50a, 50b, 50c in Figures 5A-5C, respectively, to identify the gazing patterns and attentiveness of the vehicle operator 20. The ocular profiles 50a, 50b, 50c include the position and size of the eyes 24, which is referenced generally at 52a, 52b, 52c and the corners of the eyes 24, which is referenced generally at 54a, 54b, 54c. In the following description, the ocular profile 50a is associated with an attentive vehicle operator 20 because the vehicle operator's eyes 24 are fixed on a 'forward view' of the road, which is generally correlated to a forward view window at reference numeral 75 in Figure 6. Using the forward view window 75 as a reference for vehicle operator attentiveness, the ocular profiles 50b, 50c, for example, are associated with a non-attentive vehicle operator 20 who has a 'non-forward' or distracted view of the road that is generally outside of the forward view window 75.

The present disclosure utilizes the identification of vehicle operator attentiveness determined from distracted, 'non-forward' ocular profiles 50b, 50c so that countermeasures can be taken to enhance the operation of vehicle systems. The real time duration and frequency of the vehicle operator's distracted, 'non-forward' view may be captured to identify if the vehicle operator 20 is distracted, if, for example, a particular task is being conducted by the vehicle operator 20, such as, for example, radio-tuning, that distracts the vehicle operator 20 from maintaining attentiveness of the road ahead in the forward view window 75.

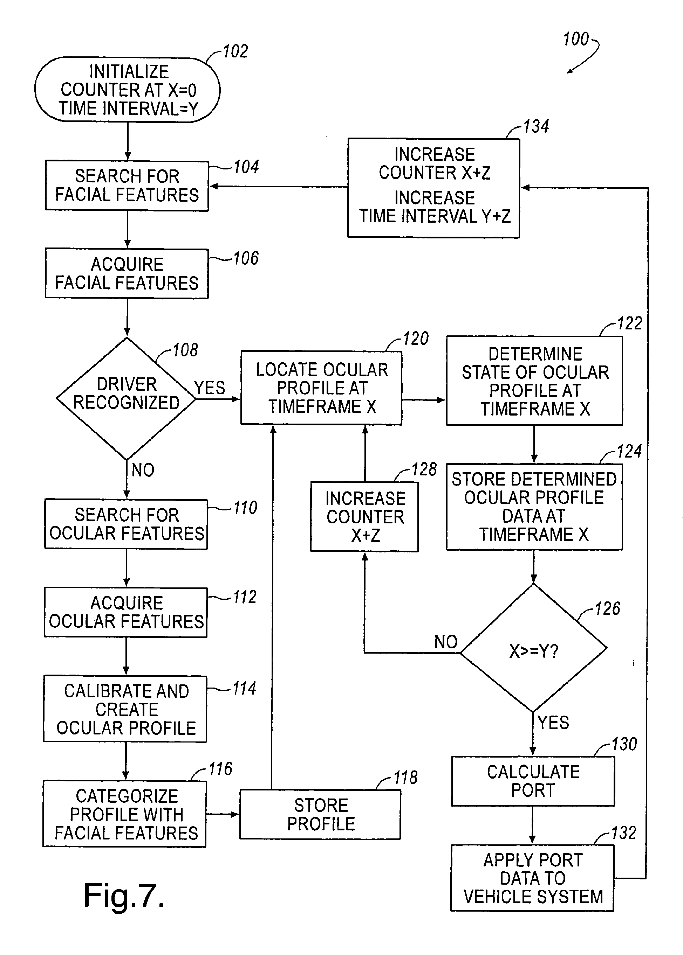

Referring to Figure 7, a real-time, data-driven method for determining the visual distraction of the vehicle operator 20 is shown generally at 100. As generally seen in steps 102-134, a series of ocular profiles 50a, 50b, 50c is captured for subsequent analysis and application to a vehicle system to maintain or adjust a state of the vehicle system in response to the real-time identification of the attentiveness of the vehicle operator 20. Steps 104-118 utilize the vehicle operator attentiveness imaging system to aquire facial and occular features of a vehicle operator 20. First, facial features are searched in step 104, and then, in step 106, the routine acquires the facial features. In a decision step 108, the routine determines if the vehicle operator 20 has been recognized. If the vehicle operator 20 has been recognized, the routine proceeds to step 120 to locate an ocular profile of the recognized vehicle operator 20. If the vehicle operator 20 has not been recognized from the facial features, the routine will search for and create a new ocular profile in steps 110 through 118. This includes searching for ocular features in step 110, acquiring ocular features in step 112, and calibrating and creating an ocular profile in step 114. In step 116, the ocular profile is categorized with facial features. Thereafter, the ocular profile is stored in memory in step 118, the routine is advanced to step 120.

Referring now to steps 120-124, a general examination of the ocular profiles 50a, 50b, 50c is conducted by locating, determining, and storing an imaged frame of the vehicle operator's ocular features at 52a-54c to determine the attentiveness type of the captured ocular profile 50a, 50b, 50c. Steps 126-128 cycle the general examination of steps 120-124 within a time interval, Y, to capture sequentially imaged frames of the ocular profile 50a, 50b, 50c to determine a proportional amount of time that a vehicle operator 20 may be classified as having an attentive ocular profile 50a or distracted ocular profile 50b, 50c. Because the examined ocular profiles 50a, 50b, 50c are captured sequentially at step 124 on a frame-rate basis, real time data can be calculated at step 130 to determine the level of vehicle operator visual distraction.

The real time data calculated at step 130 is a percentage of a series of saved data from step 124. According to an embodiment, the calculated percentage may relate to distracted ocular profiles 50b, 50c captured over a given time interval, Y. The calculation at step 130 is determined by summing frames of distracted ocular profiles 50b, 50c over the time interval, Y, and dividing the summed frames of distracted ocular profiles 50b, 50c over the time interval, Y, by a total series of frames captured over the time interval, Y, that includes attentive and distracted ocular profiles 50a, 50b, 50c. The expression for determining the calculation at step 130 is:

$$PORT = n / N$$

where "PORT" stands for Proportion of Off-Road glance Time, "n" is the number of frames that the vehicle operator's ocular profile is classified as a distracted ocular profile 50b, 50c, and "N" is a total number of predetermined series of frames (i.e. both attentive ocular profiles 50a and distracted ocular profiles 50b, 50c) to be captured over the time interval, Y.

To calculate PORT, a counter value, X, is initialized at zero and the time interval, Y, is set to any desirable value in step 102. The values of X and Y are compared at step 126. Once the value, X, of the counter is greater than or equal to the value of the time interval, Y, which is associated with the total number of frames, N, the PORT is calculated at step 130. If the criteria at step 126 is not met, the algorithm is advanced to step 128 where the counter value, X, is incremented by any desirable value, Z. This loop at steps 120-128 is continuously cycled until the criteria at step 126 is met. The value of the counter, X, time interval, Y, and incrementation value, Z, may be any desirable value, such as, for example, 0.10 seconds, 0.25 seconds, 0.50 seconds, 1 second, 2 seconds, 3 seconds, 5 seconds, 10 seconds, 15 seconds, 30 seconds, or 60 seconds.

Utilizing the calculated PORT value at step 130, a visually-distracted vehicle operator 20 may timely respond to potentially dangerous driving conditions upon application of the PORT value at step 132. PORT is found to be directly correlated with vehicle operator distraction and safety variables such as brake reaction times, lane departures, and standard deviation of lane position; as PORT increases, reaction times, lane departures, and standard deviations of lane position all increase. Accordingly, the relationship between PORT and safety criteria is reliable across a wide range of conditions. As such, PORT can be implemented in several manners to mitigate visual distraction and enhance vehicle operator safety.

According to an embodiment, a PORT threshold value can be established at step 132 as a reference value to determine when there is an excessive level of vehicle operator distraction. For example, a predetermined PORT threshold value may be set at 0.50 (i.e. 50% of the captured frames include distracted ocular profiles 50b, 50c), which is used as the basis for comparing a calculated PORT value from step 130. In application, according to an embodiment, if the calculated PORT at step 130 is 0.75 (i.e. 75% of the captured frames include distracted ocular profiles 50b, 50c), the predetermined threshold value of 0.50 has been exceeded, and thus, gentle braking pattern warnings can be delivered to the vehicle's braking system at step 132 to remind the vehicle operator 20 to pay more attention to the forward view window 75.

Using the above-described braking system example, the first application of the calculated PORT information at step 132 may take place approximately at the value of the predetermined time interval value, Y, that is set at step 102 if the vehicle operator 20 is determined to be in a distracted state. Once the application takes place at step 132, the algorithm is advanced to step 134 where the counter value, X, and the time interval value, Y, are incremented by the incrementation value, Z. After the incrementation is registered at step 134, the algorithm is returned to step 104 so that the ocular profile of the vehicle operator 20 may be examined at subsequent timeframes. Accordingly, the ocular profile data for a subsequent timeframe is stored step 124, PORT is calculated for the subsequent timeframe at step 130, and the application of the PORT data is conducted once again at step 132. Upon feeling the gentle braking patterns, the vehicle operator 20 may become more attentive to the road (i.e. the vehicle operator transitions to an attentive ocular profile 50a), thereby drawing down the calculated PORT value from 0.75. As the vehicle operator 20 maintains attentiveness of the forward view window 75, the calculated PORT value eventually falls below the predetermined PORT threshold value of 0.50 and the gentle braking pattern warnings are ceased, thereby restoring normal operation of the vehicle 10.

The calculation and application of the PORT data at steps 130, 132 for subsequent timeframes may take place for any desirable period. For example, ocular profiles 50a, 50b, 50c may be examined over a running (i.e. limitless) time interval, Y. Accordingly, when the vehicle operator 20 keys the vehicle, the time interval Y may be continuously incremented until the vehicle 10 is keyed-off so that attentiveness of the vehicle operator 20 may be determined over the entire operating period of the vehicle 10. Alternatively, the calculated and applied PORT data may be utilized for recent (i.e. limited) time intervals, Y. Accordingly, the most recent, newly-captured ocular profile data may 'bump' the oldest ocular profile data such that the calculated PORT value from step 130 over a limited time interval, Y, can be constrained to the most recent ocular profile data. For example, if Y is set to be equal to 5 seconds and Z is equal to 1 second, the first PORT calculation at step 130 takes place from the time interval of 0-5 seconds; accordingly, the subsequent calculation of PORT values at step 130 may be over the limited time interval range of 1-6 seconds, 2-7 seconds, 3-8 seconds, etc. Thus, the applied PORT data at step 132 may be refined such that the PORT data is related to the most recent vehicle operator attentiveness information.

According to an embodiment, safety warning systems, such as, for example, forward collision warning (FCW) systems, can be enhanced using the calculated PORT information from step 130. As is known, FCW systems include high levels of "nuisance alerts." Because FCW system alerts are associated with the timing of a potential collision, the real-time calculated PORT information may be utilized for comparison to the threshold value in step 132 as described above to more appropriately time and reduce the number of alerts. Thus, according to an embodiment, if a calculated PORT value of 0.75 exceeds a predetermined PORT value of 0.50, step 132 may modify the alert sent to the FCW system so that the distracted vehicle operator 20 may receive the alert earlier than normal to provide additional time for the vehicle operator 20 to avoid a crash. Conversely, if the calculated PORT value is 0.10 (i.e. 10% of the captured frames include distracted ocular profiles 50b, 50c), which is below the predetermined PORT value of 0.50, step 132 may apply a signal to the FCW system that maintains, disables, or modifies the alert so that a generally non-distracted vehicle operator 20 will be less likely to be annoyed by alerts since the vehicle operator's ocular profile 50a is focused on the road in the forward view window 75 approximately 90% of the time.

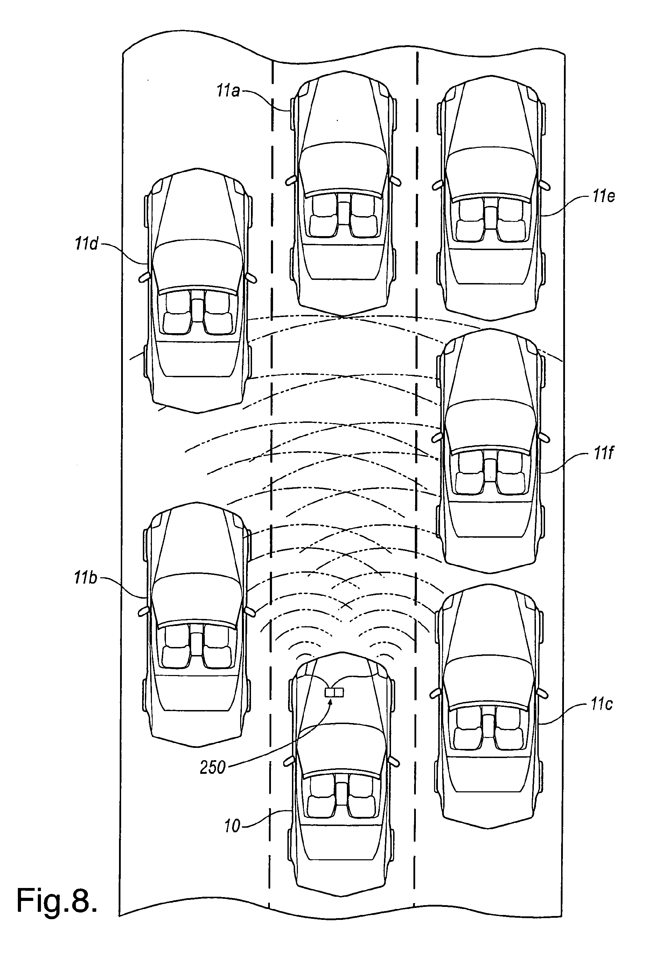

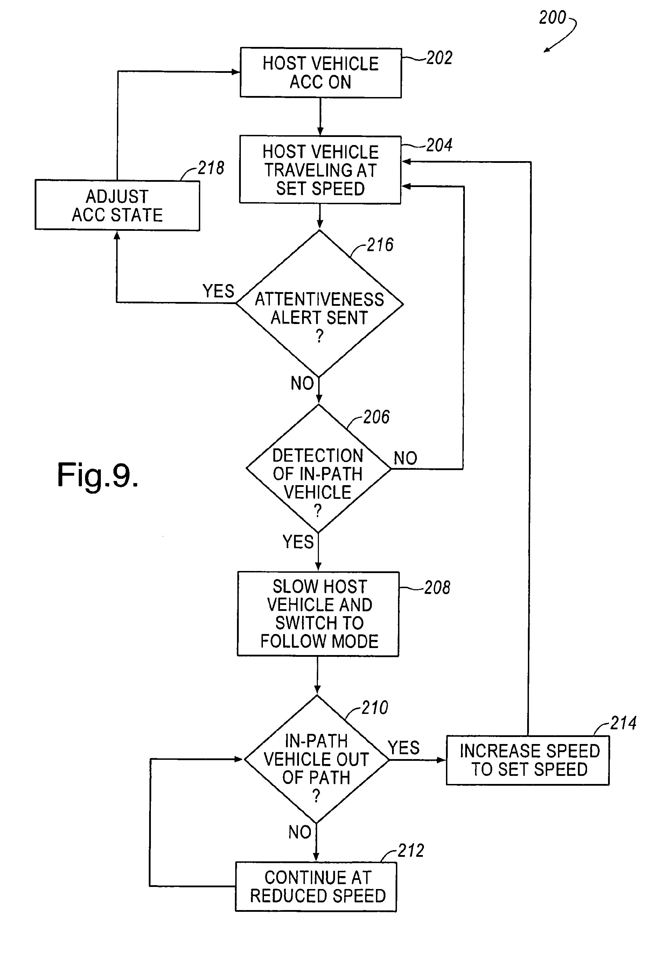

According to another embodiment, a vehicle operator attentiveness alert / calculated PORT value from step 130 may be utilized in a similar manner as described above in an automated cruise control (ACC) system, which is shown generally at 250 in Figure 8. The method for operating the ACC system 250 is shown generally at 200 in Figure 9. As is known, ACC provides to the vehicle operator 20 a convenience by maintaining a constant set speed or automatically maintaining a constant headway of a host vehicle 10 in view of an in-path vehicle / near object 11a-11f. Normal operation of the ACC is shown generally at steps 202-214. By applying vehicle operator attentiveness information or the PORT information from a vehicle operator attentiveness imaging system in the form of an alert at step 216 while the ACC is engaged, the ACC may make appropriate adjustments at step 218 to the following distance of a host vehicle 10. Accordingly, the ACC system 250 may operate under normal conditions after step 216 (i.e. step 216 is advanced to step 206) when the vehicle operator 20 maintains a forward view of the road, and, if the vehicle operator 20 becomes distracted, the ACC system 250 operates in an altered state by utilizing the vehicle operator awareness data (i.e. step 216 is advanced to step 218). Upon adjusting the ACC of the vehicle 10 at step 218, the ACC method 200 is then cycled back to the on-state of the ACC system at step 202; however, if desired, the adjusted ACC state at step 218 may alternatively result in the subsequent deactivation of the ACC system.

In application, the vehicle operator attentiveness alert / PORT information from step 216 is used at step 218 as feedback for adjusting the ACC system 250 based on the alertness level of the vehicle operator 20. According to an embodiment, adjustments can be made to the ACC system at step 218 to increase the following distance (i.e., by reducing throttle) to allow for longer reaction time when a vehicle operator 20 is distracted. In an another embodiment, the ACC system 250 may apply the brakes at step 218 in response to slower-moving or stationary vehicle / objects l la-1 If during periods of vehicle operator distraction. The braking at step 218 may be applied to the vehicle 10 in a manner to alert the vehicle operator 20 so as to acquire vehicle operator attention quickly. For example, a low automatic braking may provide a very powerful stimulus for returning the vehicle operator's attention to a forward view window 75. The adjusted ACC state at step 218 may occur for any desired period. If desired, steps 202, 204, 216, and 218 may be continuously looped until the vehicle operator maintains an attentive ocular profile 50a for an expected time interval or period.

While the invention has been specifically described in connection with certain specific embodiments thereof, it is to be understood that this is by way of illustration and not of limitation, and the scope of the appended claims should be construed as broadly as the prior art will permit.

**Claims**

1. A vehicle operator monitoring method (100) comprising the steps of:

   capturing (104-128) a series of ocular profiles (50a, 50b, 50c) of a vehicle operator (20);
   conducting an analysis (130) of the series of ocular profiles (50a, 50b, 50c) of the vehicle operator (20);
   applying the analysis (132) to a vehicle system (250) to maintain or adjust a state of the vehicle system (250).

2. The vehicle operator monitoring method (100) according to Claim 1, wherein the capuring step includes the steps of
   utilizing a vehicle operator attentiveness imaging system having a video imaging camera (16) to aquire (104-118) facial features of a vehicle operator (20);
   examining (120-124) an imaged frame of the facial features to locate an ocular profile (50a, 50b, 50c); and
   cycling (126-128) the capturing step within a time interval to examine sequentially captured imaged frames of the ocular profile (50a, 50b, 50c) to create the series of ocular profiles (50a, 50b, 50c).

3. The vehicle operator monitoring method (100) according to Claim 2, wherein the examining step (120-124) further comprises the steps of
determining (122) if the ocular profile is related to an attentive vehicle operator ocular profile (SOa) or a distracted vehicle operator ocular profile (50b, 50c); and
storing (124) data associated with the determining step in memory.

4. The vehicle operator monitoring method (100) according to Claim 3, wherein the conducting an analysis step further comprises the step of calculating (130) a proportion of off-road glance time by dividing the distracted vehicle operator ocular profiles (50b, 50c) associated with the stored data over a total number of attentive vehicle operator ocular profiles (50a) and the distracted vehicle operator ocular profiles (50b, 50c) associated with the stored data.

5. The vehicle operator monitoring method (100) according to Claim 4, wherein the applying the analysis step (132) further comprises the step of
altering the state of the vehicle system (250) based upon a comparison of the proportion of off-road glance time and a predetermined threshold value.

6. The vehicle operator monitoring method (100) according to Claim 5, wherein the vehicle system (250) is an automated cruise control system.

7. The vehicle operator monitoring method (100) according to Claim 6, wherein the altering step includes reducing throttle of a host vehicle (10) to increase the following distance of the host vehicle (10) in view of an in-line vehicle/object (l la-l lf) when a vehicle operator (20) is distracted.

8. The vehicle operator monitoring method (100) according to Claim 6, wherein the altering step includes applying the brakes of a host vehicle (10) to increase the following distance of the host vehicle (10) in view of an in-line vehicle/object (11a-11f) when a vehicle operator (20) is distracted.

9. The vehicle operator monitoring method (100) according to Claim 5, wherein the vehicle system is a forward collision warning system.

10. The vehicle operator monitoring method (100) according to Claim 9, wherein the altering step includes receiving an alert earlier than normal when a vehicle operator (20) is distracted.

11. The vehicle operator monitoring method (100) according to Claim 10, wherein the altering step includes disabling or modifying the alert when the vehicle operator (20) is attentive.

**12.** A vehicle operator monitoring system, comprising:

an automated cruise control system (250) that regulates the following distance of a host vehicle (10) in view of an in-line vehicle / object (11a-11f); and

a vehicle operator attentiveness imaging system that monitors the alertness level of a vehicle operator (20) inside a passenger compartment (12) of a vehicle (10), wherein an alert signal from the vehicle operator attentiveness imaging system is applied to the automated cruise control system (250) for adjusting a state of the automated cruise control system (250) based on the alertness level of the vehicle operator (20) determined by the vehicle operator attentiveness imaging system.

**13.** The vehicle operator monitoring system according to Claim 12, wherein, upon determining the vehicle operator (20) is distracted, the alert signal disables the automated cruise control system (250).

**14.** The vehicle operator monitoring system according to Claim 12, wherein, upon determining the vehicle operator (20) is distracted, the alert signal causes the automated cruise control system (250) to increase the following distance by reducing throttle of the host vehicle (10).

**15.** The vehicle operator monitoring system according to Claim 12, wherein, upon determining the vehicle operator (20) is distracted, the alert signal causes the automated cruise control system (250) to increase the following distance by braking the host vehicle (10).

**16.** The vehicle operator monitoring system according to Claim 12, wherein, upon determining the vehicle operator (20) is distracted, the alert signal causes the automated cruise control system (250) to stimulate attention of the vehicle operator (20) by providing a low automatic braking to the host vehicle (10).

Fig.1.

Fig.2.

Fig.3.

Fig.4.

EP 1 723 901 A1

Fig.5A.

Fig.5B.

Fig.5C.

Fig.6.

*100*

```
┌──────────────┐
│  INITIALIZE  │  ┌─102
│ COUNTER AT X=0│
│TIME INTERVAL=Y│
└──────┬───────┘
       │
       ▼
┌──────────────┐     ┌─104        ┌──────────────────┐  ┌─134
│  SEARCH FOR  │◄────────────────│     INCREASE      │
│FACIAL FEATURES│               │   COUNTER X+Z    │
└──────┬───────┘                │                  │
       │                        │    INCREASE      │
       ▼                        │ TIME INTERVAL Y+Z│
┌──────────────┐  ┌─106         └──────────────────┘
│   ACQUIRE    │
│FACIAL FEATURES│
└──────┬───────┘
```

INITIALIZE COUNTER AT X=0 TIME INTERVAL=Y — 102

SEARCH FOR FACIAL FEATURES — 104

INCREASE COUNTER X+Z INCREASE TIME INTERVAL Y+Z — 134

ACQUIRE FACIAL FEATURES — 106

DRIVER RECOGNIZED — 108

YES

LOCATE OCULAR PROFILE AT TIMEFRAME X — 120

DETERMINE STATE OF OCULAR PROFILE AT TIMEFRAME X — 122

NO

SEARCH FOR OCULAR FEATURES — 110

INCREASE COUNTER X+Z — 128

STORE DETERMINED OCULAR PROFILE DATA AT TIMEFRAME X — 124

ACQUIRE OCULAR FEATURES — 112

CALIBRATE AND CREATE OCULAR PROFILE — 114

X>=Y? — 126

NO

YES

CATEGORIZE PROFILE WITH FACIAL FEATURES — 116

STORE PROFILE — 118

CALCULATE PORT — 130

APPLY PORT DATA TO VEHICLE SYSTEM — 132

Fig.7.

Fig.8.

_200_

HOST VEHICLE
ACC ON — _202_

HOST VEHICLE
TRAVELING AT
SET SPEED — _204_

ADJUST
ACC STATE — _218_

ATTENTIVENESS
ALERT SENT
? — _216_

YES

NO

Fig.9.

DETECTION
OF IN-PATH
VEHICLE
? — _206_

NO

YES

SLOW HOST
VEHICLE AND
SWITCH TO
FOLLOW MODE — _208_

IN-PATH
VEHICLE OUT
OF PATH
? — _210_

YES

INCREASE SPEED
TO SET SPEED — _214_

NO

CONTINUE AT
REDUCED SPEED — _212_

**European Patent Office**

**EUROPEAN SEARCH REPORT**

Application Number

EP 06 07 5964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/034905 A (VOLVO TECHNOLOGY CORPORATION; VICTOR, TRENT; LARSSON, PETTER) 29 April 2004 (2004-04-29)<br>* page 30, line 1 - line 19 *<br>* page 14, line 8 - line 12 *<br>* page 44, lines 1-6 *<br>* page 47 - page 48; figures 1-20 *<br>* claims 41-49 *<br>----- | 1-16 | INV.<br>A61B3/113 |
| X | DE 101 35 742 A1 (DAIMLERCHRYSLER AG) 27 February 2003 (2003-02-27)<br>* paragraph [0004] - paragraph [0014]; figure 1 *<br>----- | 1-16 | |
| A | WO 03/070093 A (VOLVO TECHNOLOGY CORPORATION; VICTOR, TRENT; LARSSON, PETTER) 28 August 2003 (2003-08-28)<br>* the whole document *<br>----- | 1-16 | |
| | | | TECHNICAL FIELDS SEARCHED (IPC)<br><br>A61B<br>B60K |

The present search report has been drawn up for all claims

| Place of search | Date of completion of the search | Examiner |
|---|---|---|
| Munich | 21 September 2006 | Birkenmaier, Tamara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**

EP 06 07 5964

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2006

| Patent document cited in search report | | Publication date | Patent family member(s) | | Publication date |
|---|---|---|---|---|---|
| WO 2004034905 | A | 29-04-2004 | AU | 2003269772 A1 | 04-05-2004 |
| | | | BR | 0315384 A | 06-09-2005 |
| | | | CN | 1705454 A | 07-12-2005 |
| | | | EP | 1553872 A1 | 20-07-2005 |
| | | | JP | 2006502796 T | 26-01-2006 |
| DE 10135742 | A1 | 27-02-2003 | NONE | | |
| WO 03070093 | A | 28-08-2003 | AU | 2003211065 A1 | 09-09-2003 |
| | | | BR | PI0307760 A | 04-04-2006 |
| | | | CN | 1627915 A | 15-06-2005 |
| | | | EP | 1478268 A1 | 24-11-2004 |
| | | | JP | 2005517484 T | 16-06-2005 |
| | | | US | 2003181822 A1 | 25-09-2003 |

EPO FORM P0459

For more details about this annex : see Official Journal of the European Patent Office, No. 12/82